# EUROPEAN PATENT SPECIFICATION

(11) **EP 0 477 240 B1**
(45) Date of publication and mention of the grant of the patent: **23.08.1995**
(21) Application number: 90909343.7
(22) Date of filing: 12.06.1990
(51) Int. Cl.: A61K 39/12

(54) **METHOD OF PREPARING VACCINES AGAINST RNA VIRUSES**
VERFAHREN ZUR HERSTELLUNG VON IMPFSTOFFEN GEGEN RNA-VIRUS
PROCEDE DE PREPARATION DE VACCINS CONTRE LES VIRUS LIES A L'ARN

(30) Priority: 14.06.1989 GB 8913665
(43) Date of publication of application: 01.04.1992
(73) Proprietor: THE UNIVERSITY OF BIRMINGHAM, Birmingham B15 2TJ (GB)
(72) Inventor: SKINNER, Gordon, Robert, Bruce Harborough Banks, Solihull B94 6LD (GB); BUCHAN, Alexander, Birmingham B17 0B7 (GB)
(74) Representative: Pearce, Anthony Richmond
(86) International application number: GB9000905
(87) International publication number: WO9015622

(56) References cited:
- EP-A- 0 089 854
- GB-A- 1 267 591

## Description

There is an urgent need to develop effective vaccines against a number of RNA virus infections, including in particular those induced by the RNA viruses, which include the picorna viruses such as encephalo myocarditis virus; the orthomyxoviruses such as influenza A; and the retroviruses such as mouse leukaemia/mouse sarcoma virus. The retroviruses also include human immunodeficiency disease virus (HIV) and human T-cell leukaemia virus-1 (HTLV I), which are responsible for chronic, psychologically distressing and fatal diseases. In the case of HIV there is a real possibility of a global epidemic within the next two decades.

Retroviruses are unusual in that virus replication is mediated by an intermediate stage of DNA synthesis from the input RNA by a viral enzyme, reverse transcriptase. This raises the complication that inoculation of these nucleic acids, in particular the DNA, might lead to transcription of ribonucleic acid in the human subject resulting in production of infectious virus particles or possibly oncogenic outcome. Such effects are clearly unacceptable in a proposed vaccine preparation. It is equally important that such a vaccine does not contain any whole virus particles which might be infectious, and that the vaccine shall not contain RNA which might have infectious or pathogenic potential or be transcribed into DNA, or contain DNA which might be infectious or oncogenic.

Infection by the picorna viruses and orthomyxoviruses does not involve a DNA transcription process, whereby not virus DNA is present at any stage of the infection process.

It is known from EP-A-0089854 to prepare a vaccine against a DNA virus by separating and discarding the intact nuclei of cells which have been infected with the virus. Required antigenic proteins are then separated from the remaining cytoplasmic fraction. Since over 90% of cell and virus DNA is contained within the nuclei of infected cells, the above procedure removes the larger part of that DNA without adversely affecting the antigenic proteins in the cytoplasmic fraction.

The present invention has as an object the preparation of a vaccine against a RNA virus, the vaccine being free of virus particles, synthesised virus DNA and active virus RNA.

This object is achieved by infecting cells with a RNA virus, incubating the infected cells for a time and at a temperature which is insufficient to result in cell lysis, separating the cell nuclei intact from the cell cytoplasmic fraction, introducing a fixing agent into the separated cytoplasmic fraction to stabilise the viral antigenic proteins therein, and precipitating said viral antigenic proteins, said proteins providing the active constituent of the vaccine.

One embodiment of the invention will now be described by way of example, with reference to influenza virus A, strain NWS. In the following description the term virus particle is to be understood as relating to the entire virus structure, comprising nucleic acid surrounded by a protein shell. Some viruses have an envelope surrounding the protein shell, in which case the term virus particle also includes the envelope.

Hamster kidney cells (BHK-21, Macpherson and Stoker 1962) are cultivated in Eagle's medium supplemented with 10% foetal or newborn calf serum and 10% tryptose phosphate broth, to growth in rotating Winchester bottles (2,500ml). Aliquots of the cultivated cells are stored in a glycerol-containing medium at -70°C for subsequent use.

The cells are allowed to reach near confluence as sheets within the Winchester bottles. Each cell sheet is subsequently washed with prewarmed Eagle's medium to remove the foetal calf serum. The cells are maintained in a serum-deprived condition for 24 hours. This level of serum deprivation will not result in a significant reduction in the level of virus antigens obtained as a result of the described method. Though serum deprivation has previously been considered necessary for vaccine production, in order to reduce the level of calf serum in the resulting vaccine, it is envisaged that at least some of the steps described hereafter, for precipitating virus proteins from a purified cytoplasmic fraction of the infected cells, may allow the serum deprivation step to be omitted entirely.

The aforesaid influenza virus is added to the serum-deprived cells at between 0.5 and 5 plaque-forming units per cell. The cultures are then incubated to a stage insufficient to result in cell lysis, and at which there is substantially 100% cytopathic effect. The cells are gently harvested, washed in phosphate buffered saline, and resuspended to a concentration of 2 x 10⁷ cells/ml.

Nonidet (NP-40) is added to a concentration of 1% by volume and the cells are maintained thus at 4°C for 10 minutes. The concentration and temperature of the Nonidet surfactant is insufficient to disrupt the cell nuclei, which thus remain intact, and retain substantially the whole of their contents. The Nonidet parts the cell nuclei from their cytoplasm and strips important antigenic proteins from enveloped virus particles which may be present in the cytoplasm. The Nonidet also reduces, but may not totally eliminate, the infectivity of the virus particles.

The cells are centrifuged at 400g for 10 minutes and the supernatant, which is the cytoplasmic fraction of the cell preparation is collected, the remaining material being discarded. The supernatant at this stage contains virus antigenic proteins, virus particles, BHK cell proteins and proteins from the culture serum, as well as small amounts of virus RNA and cell DNA, together with Nonidet detergent.

The polypeptide chains in the cytoplasmic fraction are stabilized by addition of a known fixing agent such as formaldehyde or glutaraldehyde for 18 hours at room temperature, to a final concentration of 0.04%. In addition to fixing the polypeptide chains this step inactivates any residual virus RNA and cell DNA, and inactivates any residual virus particles.

The resulting fraction is subjected to ultra sonication vibration by a Probe sonicator which serves to disrupt residual nucleic acid, and then centrifuged over a cushion of sucrose at a concentration of 20% W/V for 5 hours at 80,000g in a bucket of 40ml volume. The 4ml top fraction in each centrifuge container comprises a purified cytoplasmic fraction which has been found to contain no virus particles or virus nucleic acid, and to consist of virus antigenic proteins, together with cell and serum proteins, as well as the formaldehyde, sucrose and detergent previously added.

The purified cytoplasmic fraction is then subjected to a process which precipitates the proteins from the fraction, either alone or in combination with antibodies to that protein. This precipitation is preferably effected by addition of a polar organic solvent, such as acetone, ethanol or methanol, which when mixed with water lowers the dielectric constant of the mixture and increases protein-protein interactions. The solvent is added at a temperature between 0°C and -30°C, at a ratio of 10 parts of solvent to 1 part of water in the cytoplasmic fraction. Preferably the solvent temperature is between -10°C and -20°C. The precipitate is separated by centrifugation and the remaining solvent is evaporated off.

The precipitate is then redissolved in phosphate buffered saline, and freeze-dried for storage.

The vaccine preparation thus obtained has been found to be antigenic, immunogenic and substantially free of viral nucleic acid.

The cold solvent precipitation step described above precipitates all of the proteins in the purified cytoplasmic fraction, making necessary serum deprivation of the BHK cells before infection. Other separation procedures, as for example forming antigen-antibody complexes with the desired antigenic proteins, may render the serum deprivation step unnecessary.

The procedure described above is applicable to other RNA viruses for example picorna viruses (a small unenveloped virus) or retroviruses such as HIV and HTLV, where it is important to ensure that not only RNA but also DNA has been removed from the preparation. In the case of retroviruses transcription of virus RNA into virus DNA is believed to occur in the cytoplasm of infected cells, but it is known that almost the whole of the virus DNA is located within the cell nuclei very shortly after infection. The procedure of the present invention, whereby the infected cells are incubated for a time which will ensure maximum infectivity but is insufficient to result in cell lysis, has the effect that substantially no virus RNA or DNA is present in the cytoplasmic fraction of the infected cells. As described above, the subsequent fixing, ultrasonication and centrifugation steps performed on the separated cytoplasmic fraction first inactivate any residual virus RNA and DNA and any residual virus particles, disrupt any residual nucleic acids, and remove the remnants of the virus particles and nucleic acids.

## Claims

1. A method of preparing a vaccine against a RNA virus, comprising infecting cells with said virus, incubating the infected cells for a time and at a temperature which is insufficient to result in cell lysis, separating the cell nuclei intact from the cell cytoplasmic fraction, introducing a fixing agent into the separated cytoplasmic fraction to stabilise the viral antigenic proteins therein, and precipitating said viral antigenic proteins, said proteins providing the active constituent of the vaccine.

2. A method as claimed in claim 1 in which said cells are serum-deprived before infection with said virus.

3. A method as claimed in claim 1 or claim 2 in which separation of the cell nuclei from the cytoplasmic fraction is effected by treatment of said cells with a surfactant at a concentration and temperature and for a time which is insufficient to disrupt the cell nuclei, and centrifuging the treated cells.

4. A method as claimed in any preceding claim in which the cytoplasmic fraction containing stabilized antigenic proteins is subjected to ultrasonic vibration.

5. A method as claimed in claim 4 in which the ultrasonicated fraction is centrifuged to remove virus particles and to provide a purified top fraction of said cytoplasmic fraction.

6. A method as claimed in any preceding claim in which said viral antigenic proteins are precipitated by addition of a polar organic solvent which lowers the dielectric constant of said cytoplasmic fraction and increases protein-protein reactions therein.

7. A method as claimed in claim 6 in which said solvent is at a temperature between 0° and -30°.

8. A method as claimed in claim 6 or claim 7 in which the precipitated virus antigenic protein is separated from the cytoplasmic fraction by centrifugation.

9. A method as claimed in any preceding claim in which said virus is a picorna virus.

10. A method as claimed in any preceding claim in which said virus is a retro virus.

11. A method as claimed in any preceding claim in which said virus is an orthomyxovirus.

## Patentansprüche

1. Verfahren zur Herstellung eines Vakzins gegen einen RNA-Virus, das folgende Schritte umfaßt: Infektion der Zellen mit dem Virus, Inkubation der infizierten Zellen über eine Zeitspanne und bei einer Temperatur, die nicht ausreichen, um zu einer Zell-Lyse zu führen, Separation der Zellkerne intakt von der zell-zytoplasmatischen Fraktion, Einführen eines Fixierungsmittels in die separierte zytoplasmatische Fraktion, um die viralen antigenen Proteine darin zu stabilisieren, und Ausfällen der viralen antigenen Proteine, wobei die Proteine den aktiven Bestandteil des Vakzins darstellen.

2. Verfahren nach Anspruch 1, bei dem die Zellen vor der Infektion mit dem Virus einem Serumentzug unterzogen werden.

3. Verfahren nach Anspruch 1 oder Anspruch 2, bei dem die Separation der Zellkerne von der zytoplasmatischen Fraktion durch die Behandlung der Zellen mit einer oberflächenaktiven Substanz bei einer Konzentration und Temperatur und über eine Zeitdauer, welche nicht ausreichend sind, um die Zellkerne zu spalten, und durch das Zentrifugieren der behandelten Zellen ausgeführt wird.

4. Verfahren nach einem der hervorgehenden Ansprüche, bei dem die zytoplasmatische Fraktion, welche die stabilisierten antigenen Proteine enthält, einer Ultraschall-Vibrationsbehandlung unterzogen wird.

5. Verfahren nach Anspruch 4, bei dem die ultraschallbehandelte Fraktion zentrifugiert wird, um Virenpartikel zu entfernen und eine gereinigte oberste Fraktion der zytoplasmatischen Fraktion zu schaffen.

6. Verfahren nach einem der hervorgehenden Ansprüche, bei dem die viralen antigenen Proteine durch den Zusatz eines polaren anorganischen Lösungsmittels ausgefällt werden, das die Dielektrizitätskonstante der zytoplasmatischen Fraktion senkt und die Protein-Protein-Wechselwirkungen in dieser steigert.

7. Verfahren nach Anspruch 6, bei dem das Lösungsmittel eine Temperatur zwischen 0° und -30° C aufweist.

8. Verfahren nach Anspruch 6 oder Anspruch 7, bei dem das ausgefällte virale antigene Protein von der zytoplasmatischen Fraktion durch Zentrifugieren separiert wird.

9. Verfahren nach einem der hervorgehenden Ansprüche, bei dem das Virus ein Picornavirus ist.

10. Verfahren nach einem der hervorgehenden Ansprüche, bei dem das Virus ein Retrovirus ist.

11. Verfahren nach einem der hervorgehenden Ansprüche, bei dem das Virus ein Orthomyxovirus ist.

## Revendications

1. Procédé de préparation d'un vaccin contre un virus d'ARN, comprenant l'infection des cellules par ledit virus, l'incubation des cellules infectées pendant une période de temps et à une température insuffisantes pour entraîner une lyse cellulaire, la séparation des noyaux cellulaires intacts de la fraction cytoplasmique cellulaire, l'introduction d'un agent de fixation dans la fraction cytoplasmique séparée pour stabiliser les protéines virales antigéniques qui y sont contenues et la précipitation desdites protéines virales antigéniques, lesdites protéines constituant le composant actif du vaccin.

2. Procédé selon la revendication 1, dans lequel lesdites cellules sont privées de sérum avant l'infection par ledit virus.

3. Procédé selon les revendications 1 ou 2, dans lequel la séparation des noyaux cellulaires de la fraction cytoplasmique est effectuée par un traitement desdites cellules avec un surfactant à une concentration, une température et pendant une période de temps insuffisantes pour détruire les noyaux cellulaires, les cellules traitées étant ensuite centrifugées.

4. Procédé selon l'une quelconque des revendications précédentes, dans lequel la fraction cytoplasmique contenant des protéines antigéniques stabilisées est soumise à des vibrations ultrasoniques.

5. Procédé selon la revendication 4, dans lequel la fraction soumise aux vibrations ultrasoniques est centrifugée pour éliminer les particules virales et produire une fraction supérieure purifiée de ladite fraction cytoplasmique.

6. Procédé selon l'une quelconque des revendications précédentes, dans lequel lesdites protéines virales antigéniques sont précipitées par addition d'un solvant organique polaire, abaissant la constante diélectrique de ladite fraction cytoplasmique et accroissant les réactions protéine-protéine correspondantes.

7. Procédé selon la revendication 6, dans lequel ledit solvant a une température comprise entre 0° et -30°.

8. Procédé selon les revendications 6 ou 7, dans lequel la protéine virale antigénique précipitée est séparée de la fraction cytoplasmique par centrifugation.

9. Procédé selon l'une quelconque des revendications précédentes, dans lequel ledit virus est un virus picorna.

10. Procédé selon l'une quelconque des revendications précédentes, dans lequel ledit virus est un rétrovirus.

11. Procédé selon l'une quelconque des revendications précédentes, dans lequel ledit virus est un orthomyxovirus.
